## Europäisches Patentamt
## European Patent Office
## Office européen des brevets

(19)

(11) Publication number: **0 018 684**
**B1**

(12) **EUROPEAN PATENT SPECIFICATION**

(45) Date of publication of patent specification: **14.03.84**

(51) Int. Cl.³: **A 41 B 13/02**

(21) Application number: **80200365.7**

(22) Date of filing: **22.04.80**

(54) Disposable absorbent structure having a textured macroscopically perforated thermoplastic film topsheet.

(30) Priority: **04.05.79 US 36253**

(43) Date of publication of application:
**12.11.80 Bulletin 80/23**

(45) Publication of the grant of the patent:
**14.03.84 Bulletin 84/11**

(84) Designated Contracting States:
**BE DE FR GB IT NL**

(56) References cited:
**FR - A - 2 301 186**
**US - A - 3 292 619**
**US - A - 3 645 264**
**US - A - 3 929 135**

(73) Proprietor: **THE PROCTER & GAMBLE COMPANY**
**301 East Sixth Street**
**Cincinnati Ohio 45202 (US)**

(72) Inventor: **Sorensen, Eugene Robert**
**591 Meadowcrest Road**
**Springfield Twp., Ohio 45231 (US)**

(74) Representative: **Gibson, Tony Nicholas**
**Procter & Gamble (NTC) Limited Whitley Road**
**Longbenton Newcastle upon Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Disposable absorbent structure having a textured macroscopically perforated thermoplastic film topsheet

Background of the invention

Disposable absorbent articles are well known in the prior art and include such articles as diapers, bandages, catamenials, and the like. Such articles generally comprise s fluid permeable topsheet overlaying an absorbent element. Many topsheet variations are taught in the prior art ranging from fibrous webs which may be woven, non-woven, or carded to perforated plastic films. The number of perforated plastic film topsheets described in the prior art alone is significant. For example, U.S. Patent 3,292,619 entitled Absorbent Dressing which issued to V. C. Egler on December 20, 1966 and U.S. Patent 3,307,545 entitled Non-Adherent Dressing which issued to P. Surowitz on March 7, 1967, both teach disposable absorbent bandages having perforated plastic film · topsheets. In addition, U.S. Patent 3,543,750 entitled Perforate Film Dressing Method having Tapered Capillaries which issued to H. Thompson on December 30, 1975 likewise teaches disposable absorptive structures having a perforated plastic topsheet.

As disclosed in the foregoing patents, the use of plastic film topsheets provide advantages not available with fibrous topsheets. Fibrous topsheets, however, have a clothlike feel and appearance which is aesthetically pleasing and desirable in many disposable absorbent articles such as in disposable diapers. Plastic film topsheets, on the other hand, tend to be characterized by a high degree of gloss and smoothness. These characteristics are so closely associated with plastic films that a sheet of material may be readily identified as a plastic film by its shine and texture.

Various methods have been suggested for reducing the gloss the smoothness of plastic films to give them a more clothlike appearance. U.S. Patent 2,304,632 entitled Wrinkled Surface Article which issued to C. R. Faelten on December 8, 1942 teaches molded plastic articles having irregularly rectangular shaped ridges disposed over the surface to produce what may be called a waved surface. In addition, U.S. Patent 2,575,046 entitled Process for Producing Ornamental Plastic Films which issued to M. A. Chavannes et al on November 13, 1951 teaches a plastic film at least part of surface of which is undulated and exhibits a scintillating effect.

In spite of the foregoing teachings, however, the manufacturer of · disposable absorbent articles heretobefore had to choose between using a topsheet which provided an aesthetically pleasing clothlike appearance and a perforated plastic film topsheet which had superior performance characteristics but which exhibited a high degree of gloss and smoothness. The prior art topsheets can be manufactured from a perforated plastic film having reduced gloss and smoothness.

It is therefore an object of the present invention to provide a disposable absorbent article having a perforated plastic film topsheet which exhibits a clothlike feel and appearance.

This and other objects of the invention will be readily apparent when considered in reference to the following description and when taken in connection with the accompanying drawings.

Summary of the invention

According to the present invention, in a disposable absorbent structure known *per se* and comprising an absorbent body, a fluid impermeable backsheet underlying the body and a macroscopically perforated thermoplastic film forming a topsheet overlying the body, the body being encased by the backsheet and the topsheet, there is provided, over substantially the entire outer surface of the perforated thermoplastic film, microscopic nubbles dimensioned so as to leave the profile of the macroscopic perforations substantially unchanged.

In addition to being textured and perforated, the topsheet may also be embossed/debossed. Embossing/debossing substantially alters the macroscopic profile of the thermoplastic film while texturing does not.

Description of the drawings

Figure 1 is a perspective view of a disposable diaper incorporating the present invention.

Figure 2 is a sectional view taken along line 2—2 of Figure 1.

Figure 3 is a greatly enlarged edge view representation of the topsheet of the present invention.

Figure 4 is a perspective schematic view of a preferred apparatus for manufacturing the topsheet of the present invention.

Figure 5 is a fragmentary, enlarged scale size elevational view of the debossing/embossing and perforating cylinder portion of the apparatus shown in Figure 4.

Figure 6 is an enlarged scale, longitudinal sectional view of the debossing/embossing and perforating cylinder of the apparatus shown in Figure 4.

Figure 7 is an end view of the debossing/embossing and perforating cylinder shown in Figure 6.

Figure 8 is a greatly enlarged scale, fragmentary portion of the end view of the debossing/embossing and perforating cylinder shown in Figure 6.

Figure 9 is a perspective view of the perforated tubular · member of the debossing/embossing and perforating cylinder shown in Figures 6—8, inclusive.

Figure 10 is an enlarged scale, fragmentary view of the radially outwardly facing surface of the perforated tubular member shown in Figure 9.

Figure 11 is an enlarged scale, fragmentary sectional view taken along line 11—11 of Figure 10.

Description of the preferred embodiment

Referring now to the drawings, there is shown a preferred embodiment of the present invention in the form of a disposable diaper intended to be worn by an infant. It should be understood, however, that the absorbent structure of the present invention is also applicable in catamenial pads, bandages, incontinent pads and briefs, and the like. As used herein, the term "disposable absorbent structure" refers to articles which are intended to absorb liquids such as body exudates, and further, which are intended to be discarded after a single use (i.e. they are not intended to be laundered or otherwise restored and then reused).

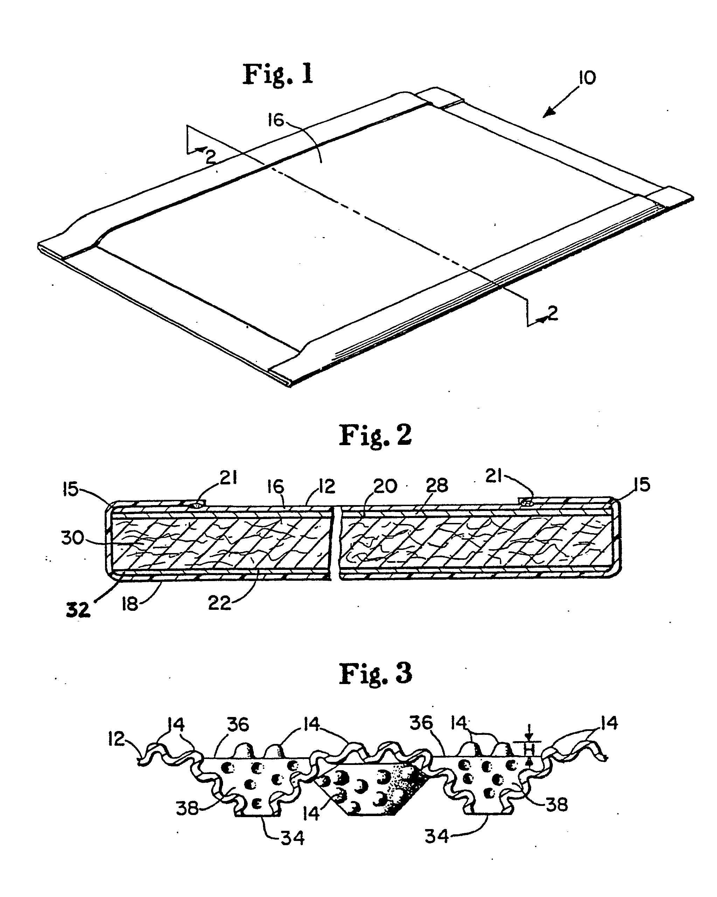

Figure 1 shows a disposable diaper 10 prior to its being applied to a diaper wearer (not shown). As best seen in Figure 2, a preferred disposable diaper 10 comprises a fluid pervious topsheet 16, a fluid impervious backsheet 18, and an absorbent layer 30. Topsheet 16 and backsheet 18 are superposed on first and second opposed faces 20 and 22, respectively, of absorbent layer 30. Topsheet 16 may be integral with or may be affixed to backsheet 18 in any suitable manner and in any configuration as is well known in the diaper art. In the preferred embodiment, for example, backsheet 18 is folded onto and overlays longitudinal side portions 15 of topsheet 16 and is affixed thereto along longitudinal seams 21, using a suitable hot melt adhesive as is commonly used and well known in the disposable diaper art. An acceptable hot melt adhesive is sold by National Starch Company of Bridge Water, New Jersey under the tradename Instant Lok 34-2933. A more detailed description of how topsheet 16, absorbent layer 30, and backsheet 18 may be assembled into a disposable diaper is given in U.S. Patent Re. 26,151 entitled Disposable diaper which issued to Robert C. Duncan et al on January 31, 1967.

Impervious backsheet 18 is preferably a 0.001 to 0.002 inch (0.025 to 0.051 mm) thick polyethylene film, although other flexible, liquid impervious materials may also be used. As used herein, flexible refers to materials which are compliant and which readily conform to the shape and contours of the human body.

Still referring to Figure 2, it can be seen that absorbent layer 30 is positioned between and encased by topsheet 16 and backsheet 18. Absorbent layer 30 may be manufactured in a wide variety of sizes and from a wide variety of absorbent materials commonly used in disposable absorbent articles capable of absorbing and retaining liquids such as absorbent foams. Other materials can also be used for absorbent layer 30 such as multiplicity of plies of creped cellulose wadding or any equivalent material. The absorbent capacity of the material used must be sufficient to meet the expected loading in the intended use of the absorbent article. In the preferred embodiment of a disposable diaper, about 48 grams of comminuted wood pump generally referred to as airfelt was used for absorbent layer 30.

First and second facing tissues 28 and 32, respectively, are superposed on, and are essentially coextensive with, first and second opposed faces 20 and 22, respectively, of absorbent layer 30. First and second facing tissues 28 and 32 help hold absorbent layer 30 in place and prevent lumping of absorbent layer 30. Preferred first and second facing tissues 28 and 32 have a basis weight of about 12 pounds per 3,000 sq.ft. (19 gms. per sq. meter) and an air permeability of about 100 cubic ft. per minute per sq.ft. (30.5 cubic meters per minute per sq.meter) over a 0.5 inch (12.7 mm.) water pressure drop and may be affixed to topsheet 16 and backsheet 18 as necessary to prevent shifting in use by any suitable method such as by gluing.

The size and shape of absorbent layer 30 may be varied to accommodate wearers ranging from infants to adults. The preferred embodiment illustrated in Figure 2 is intended for infants and absorbent core 12 is therefore of a rectangular shape approximately 12-1/2 inches (31.8 cm.) wide and 16 inches (40.6 cm.) long. Other shapes such as hourglass and trapezoidal configurations may also be used.

Porous topsheet 16 contacts the diaper wearer's skin and is therefore preferably compliant, soft feeling, fluid permeable, and non-irritating to the wearer's skin. More specifically, topsheet 16 is manufactured from a thermoplastic film which is perforated, and in which the perforations may additionally be formed by debossing/embossing. A preferred embossed and perforated topsheet 16 is shown in U.S. Patent 3,929,135 entitled Absorptive Structure having Tapered Capillaries, issued to Hugh A. Thompson on December 30, 1975. As described therein, topsheet 16 is a low density, polyethylene film having a thickness of from 0.001 to 0.002 inches (0.025 to 0.051 mm) although films having a thickness of less than 0.005 inches (0.0127 mm.) may also be used. The preferred topsheet 16 (see Figure 3) is formed to a thickness of 0.015 inches (0.38 mm.) with an array of tapered capillary structures 38 each having a perforated apex 34 and a base opening 36.

Still referring to Figure 3, it can be seen that topsheet 16 has a plurality of nubbles 14 which are integral therewith and which impart an irregular and unsmooth texture to outer surface 12 of topsheet 16. For purposes of illustration, the size of nubbles 14 are greatly exaggerated. Outer surface 12 is that surface of topsheet 16 which contacts the skin of the wearer of the absorbent article (i.e., the surface not in contact with absorbent core 30). The nubbles 14 are small protuberances projecting outward from outer surface 12 and reduce the gloss of topsheet 16 and give topsheet 16 a more clothlike tactile impression. Further, nubbles 14 do not substantially change the macroscopic profile of topsheet 16. Accordingly, topsheet 16 with nubbles 14 has a reduced plastic feel and appearance compared to thermoplastic film topsheets 16 without nubbles 14.

The number, size, and spacing of nubbles 14 may be varied within a critical range (hereinafter set forth) to give differing degrees of irregularity to outer surface 12. Nubbles 14 are preferably spherical or spheroidal in cross-section having a rounded rather than a sharp angular cross-section, although other cross sectional shapes may be used. Nubbles 14 having a maximum cross sectional dimension of from 0.0005 to 0.0110 inches (0.0127 to 0.279 millimeters) have been formed in a topsheet 16 and have been found to be satisfactory in reducing the plastic feel and impression as well as the gloss of topsheet 16. Preferably, nubbles 14 have a diameter of from 0.001 to 0.007 inches (0.025 to 0.178 millimeters) and most preferably from 0.001 to 0.005 inches (0.025 to 0.127 millimeters). The height H of nubbles 14 is measured from outer surface 12 to the top of nubbles 14 and is at least 30% of the cros-sectional diameter.

The cross-sectional diameter of the nubbles 14 referred to hereinbefore is the average diameter of a representative number of nubbles 14 and may be determined in accordance with the following procedure. A 2 inch square (5 cm square) sample of topsheet 16 is placed under a microscope having a calibrated eye piece such as is manufactured by Bausch & Lomb of Rochester, N.Y. and marketed under the tradename Stero Zoom 7 Microscope Model No. 31-26-30-07. The diameters of nubbles 14 on the sample of topsheet 16 are visually measured and the average determined.

The same procedure as hereinbefore outlined may be used to determine the average spacing between nubbles 14. Average spacing as herein used refers to the center to center distance between individual nubbles 14. A satisfactory reduction in plastic feel and appearance is obtained when nubbles 14 have an average spacing of from 1 to 6 times the maximum cross-sectional dimension. Preferably, nubbles 14 are spaced from 2 to 4 diameters apart and most preferably from 2 to 3 diameters apart.

Nubbles 14 may be formed on topsheet 16 in any of a number of ways which will suggest themselves to one skilled in the plastic film forming arts.

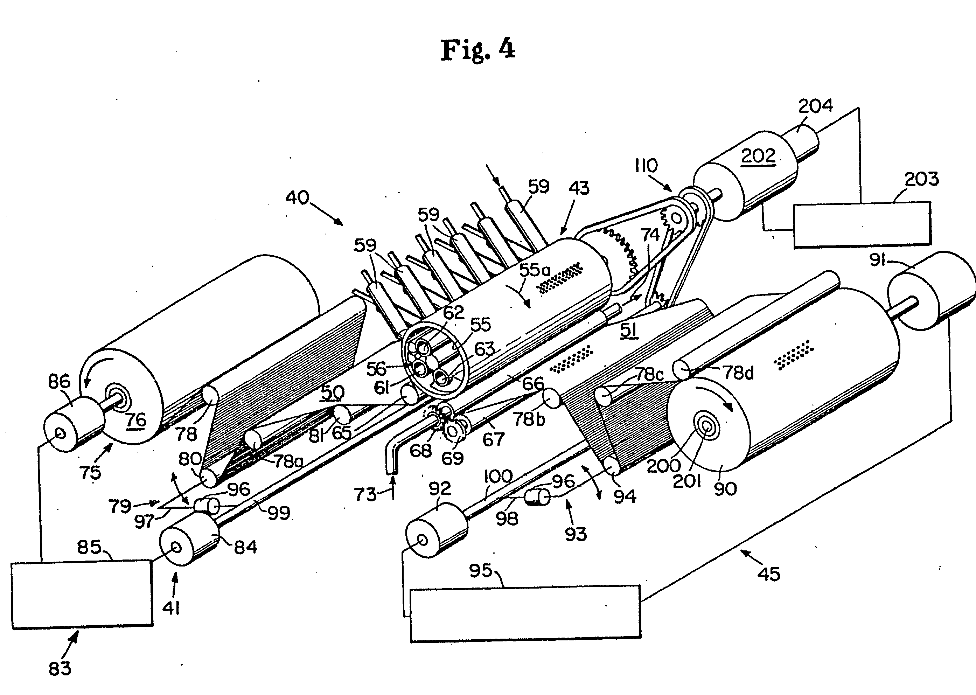

A preferred apparatus 40 is shown in Figure 4 to include constant tension film supply means 41, texturing means 43, and constant tension film forwarding and winding means 45.

The frame, bearings, supports and the like which must necessarily be provided with respect to the functional members of apparatus 40 are not shown in the figures or described in detail in order to simplify and more clearly depict and disclose the present invention, it being understood that such details would be obvious to persons of ordinary skill in the art of designing thermoplastic film converting machinery. Further, except for the texturing means 43, apparatus 40 is substantially identical to the apparatus disclosed in Lucas et al U.S. Patent No. 4,151,240, issued April 24, 1979.

Briefly, apparatus 40, Figure 4 comprises means for continuously converting a ribbon of thermoplastic film 50 into a textured film 51 by directing hot air jets against one surface of the film while applying vacuum adjacent the opposite surface of the film, and while maintaining sufficient control of the film 50 to substantially obviate wrinkling and/or macroscopically distending the film. Thus, as will be more fully described hereinafter, apparatus 40 comprises means for maintaining constant machine direction tension in the film both upstream and downstream of a zone where the temperature of the film is greater than the thermoplastic temperature of the film but in which zone there is substantially zero machine-direction and transverse-machine direction tension tending to macroscopically distend the film. The tension is required to control and smooth a running ribbon of thermoplastic film; the zero tension zone results from the film in the zone being at a sufficiently high temperature to enable texturing it through the use of heat and vacuum. Figure 4 also shows greatly enlarged scale nubbles in film 51 to enable visually perceiving the nature of the difference between the smooth film 50 and the textured film 51 as more fully described hereinafter.

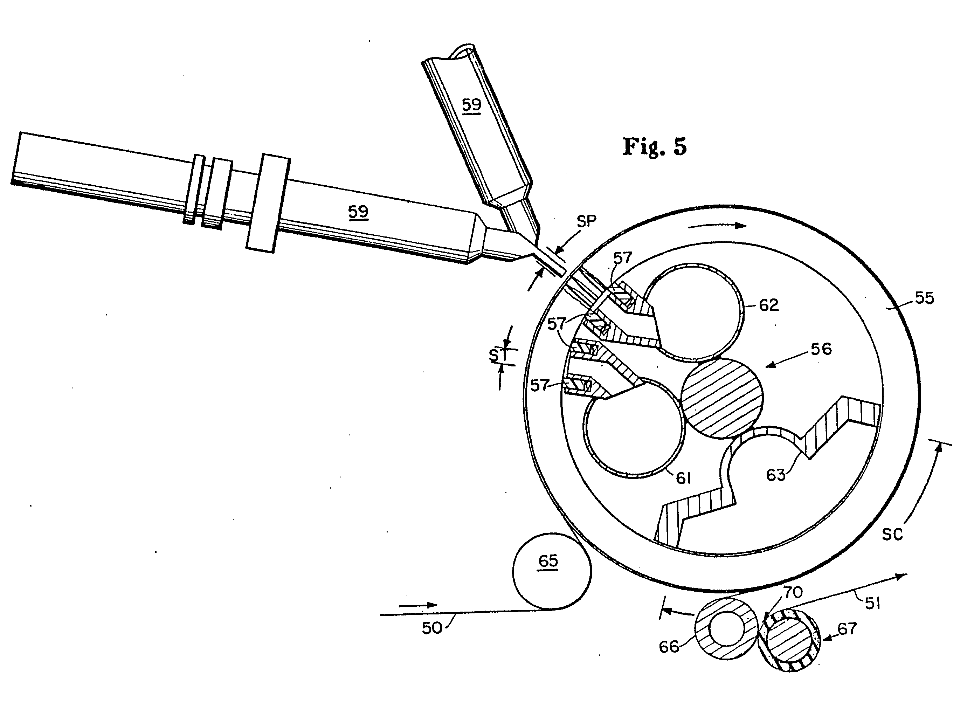

Figure 5 is an enlarged scale end view of the texturing means 43, Figure 4, which includes a rotatably mounted texturing cylinder 55, a non-rotating triplex vacuum manifold assembly 56 including seals 57, and hot air jet heaters 59. The triplex vacuum manifold assembly 56 comprises three manifolds designated 61, 62, and 63. Figure 5 also shows a freely rotatably mounted lead-on idler roll 65, a power rotated lead-off/chill roll 66, and a soft-face (e.g., low density neoprene) roll 67 which is gear driven by the chill roll through gears 68, 69, Figure 4. Briefly, by providing means (not shown) for independently controlling the degree of vacuum in the three vacuum manifolds, 61, 62, and 63, a thermoplastic ribbon of film running circumferentially about a portion of the texturing cylinder 55 is sequentially subjected to a first level of vacuum in a first sector ST, Figure 5, and a second level of vacuum in a second sector SP and a third level of vacuum in a third sector SC. As will be described more fully hereinafter, the vacuum applied to the film 50 in sector ST enables maintaining upstream tension in the film 50, vacuum in sector SP urges the film 50 against the texturing cylinder 55 and enables texturing, embossing/debossing and perforating the film when hot air is directed radially inwardly against the film 50, and vacuum in sector SC enables cooling the film 51 to below its thermoplastic temperature and enables establishing downstream tension therein. The nip 70 intermediate chill roll 66 and the soft-face roll 67 is only nominally loaded because high pressure would iron-out the debossments/embossments which are alternatively designated tapered capillaries 38 (Figure 3). However, even nominal pressure in nip 70 helps the vacuum in sector SC to isolate downstream tension (e.g., roll winding tension) from the texturing sector SP of the texturing cylinder 55, and enables the nip 70 to peel the debossed and perforated film from the texturing cylinder 55. Moreover, while vacuum drawn ambient air passing through the film in sector SC will normally cool the film to below its thermoplastic temperature, the passage of coolant through the chill roll as indicated by arrows, 73, 74 in Figure 4 will enable the apparatus to handle, for instance, thicker films, or be operated, for instance, at higher speeds.

Referring back to Figure 4, the constant tension film supply means 41 and the constant tension film forwarding and winding means 45 are substantially identical to and function substantially identically to the corresponding portions of the apparatus shown and described in U.S. Patent 3,647,221 entitled Dynamic Stress-Strain Testing of Ribbons of Film, which issued on July 4, 1972 to Coenraad E. Riemersma.

Briefly, however, the constant tension film supply means 41 comprises means 75 for rotatably mounting a roll 76 of thermoplastic film, a first idler roll 78, a dancer assembly 79 having a swingably mounted freely rotatable dancer roll 80, a second idler roll 78a, a Mount Hope Vari-Box (Registered Trademark of Mount Hope Machinery Company, Taunton, Massachusetts 02780) smoothing roll 81, the fimlead-on idler roll 65, and feedback film tension control means 83 comprising a dancer elevation position sensor 84, an unwind tension controller 85, and a variable-torque unwind brake 86. When the film 50 is looped from the roll 76 to the texturing cylinder 55 as shown in Figure 4, the film 50 is urged against and adhered to the first section ST, Figure 5, of the texturing cylinder 55 by vacuum applied through manifold 61. Thus, the film 50 is pulled as the texturing cylinder 55 rotates clockwise as indicated by the arrow 55a, while the tension control loop causes the unwind brake 86 to sufficiently resist unwinding the roll 76 of film 50 to establish and maintain a constant predetermined level of tension in the film in the machine direction. This, in turn, enables the film 50 to be tensioned transversely as it is drawn over the Mount Hope Vari-Bow roll 81 whereby it is smoothed; i.e., made wrinkle free by having a transverse tension developed in the film as it runs over the arcuate-shaped roll 81.

The tension in the film is reduced to zero, and the film 50 is textured, debossed/embossed and perforated as it passes over the second sector SP, Figure 5, of the rotating texturing cylinder 55. Then, the film is urged against and adhered to the third sector SC of the texturing cylinder 55 by vacuum applied to manifold 63 to enable the constant tension film forwarding and winding means 45 to again establish and maintain sufficient uniform tension in the film 51 to forward the film downstream under sufficient control to, for instance, wind the film to form a roll 90 of textured, debossed/embossed and perforated film. In this event, the torque of the winding drive motor 91 is controlled by the dancer-roll-position-responsive sensor 92 connected via trapeze 93 to dancer roll 94 through the winding drive and tension controller 95 to establish and maintain a substantially constant pre-determined level of machine direction tension in film 51. To summarize, the first and third vacuum sectors, ST and SC respectively, of the texturing cylinder 55 enable substantially constant upstream and downstream tension to be maintained in a running ribbon of film while the intermediate portion of the film adjacent the second vacuum sector SP of the texturing cylinder 55 is subjected to tension vitiating heat and vacuum to effect texturing, debossing/embossing and perforating the film.

Weights 96, Figure 4, on the dancer trapeze arms, 97 and 98, enable independently adjusting the upstream and downstream levels of tension: higher tensions by placing the weights 96 further from the dancer shafts 99, 100; and lower tensions by moving the weights 96 towards the dancer shafts 99, 100.

Referring again to Figure 4, the texturing means 43 comprises the rotatably mounted texturing cylinder 55, means 110 for rotating the texturing cylinder 55 at a controlled peripheral velocity, the non-rotating triplex vacuum manifold assembly 56 inside the texturing cylinder 55, means (not shown) for applying controlled levels of vacuum inside the three vacuum manifolds 61, 62 and 63 comprising the triplex manifold assembly 56, and a plurality of air heaters 59.

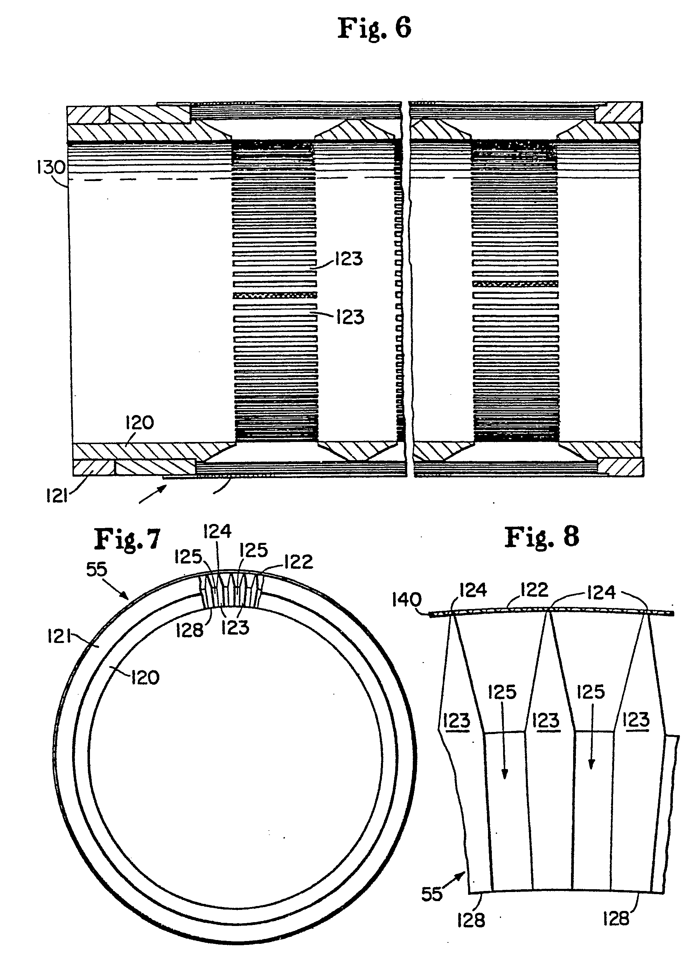

The texturing cylinder 55, Figures 6 and 7, comprises a cage 120, a support ring 121 and a thin wall perforated tubular member 122. The cage 120 comprises a multiplicity of circumferentially spaced, longitudinally extending bars 123 which are tapered to relatively small, radially outwardly facing lands 124, and the spaced bars 123 have vacuum communicating passageways 125 provided therebetween. The bars 123 also have radially inwardly facing lands 128 which cooperately provide a cylindrical vacuum sealing surface against which the vacuum seals 57, Figure 5, are biased. Thus, as the texturing cylinder 55 rotates, Figure 5, its vacuum sealing surface slides over the seals 57 of the non-rotating triplex vacuum manifold assembly 56.

The end 130, Figure 6, of the texturing cylinder 55 disposed remotely from its driven end is open in order to provide easy insertion/removal of the triplex vacuum manifold assembly 56. Therefore, in order to rotatably support the open end 130 of the texturing cylinder 55, it is provided with a bearing-race support ring 121, Figure 6, which rides on bearings, not shown, which are appropriately secured to the apparatus frame, now shown.

Figure 8 shows the perforated tubular member 122 to comprise a thin wall 140 in co-tacting relation with the small lands 124 of the cage 120. The lands 124 are small and the tubular member 122 is thin-walled because the preferred embodiment apparatus 40, Figure 4, is configured to texture, deboss/emboss and perforate a relatively thin thermoplastic film such as low density polyethylene film, thereby providing small tapered capillaries 38 as well as nubbles 14, Figure 3.

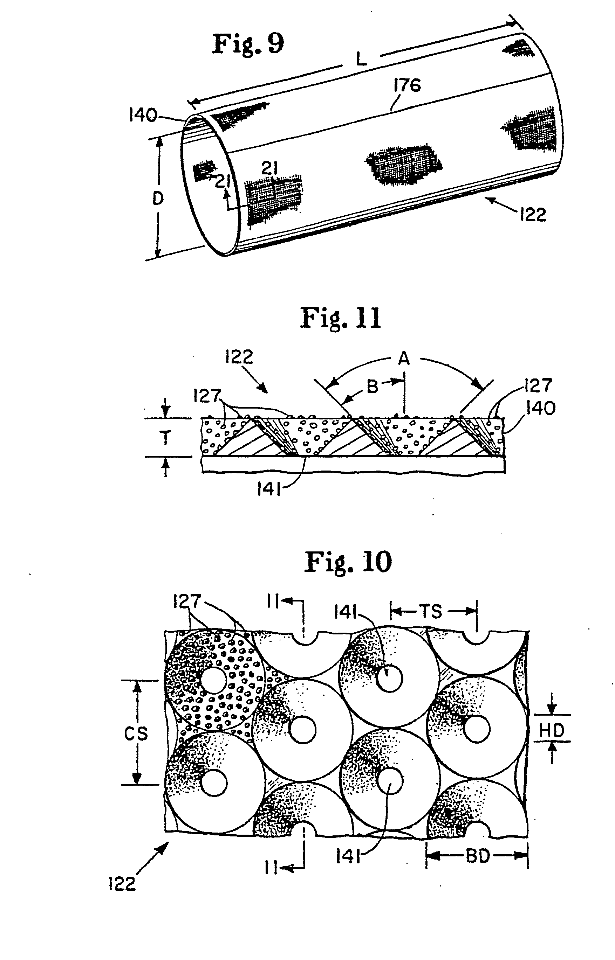

Figure 9 is a perspective view of the perforated tubular member 122 of the texturing cylinder 55 having a diameter D and length L. In the preferred embodiment, D is 8.914 inches (22.64 centimeters) and L is 21.50 inches (54.61 centimeters). Also, the member has a longitudinally extending seam, 176.

Figure 10 is a radially inwardly facing, enlarged scale view of a fragmentary portion of the perforated tubular member 122. Figure 11 is a further enlarged scale sectional view taken along line 11—11 of Figure 10. Thus, the perforated tubular member 122 of the preferred embodiment apparatus 40 comprises a thin wall 140 having a thickness T (from .013 inches (0.330 mm) to .014 inches (0.355 mm)), and having a closely packed array of tapered holes 141 therethrough. The holes 141 Figure 10, are circumferentially spaced (CS) .040 inches (0.102 centimeters) center-to-center, transversely spaced (TS) .0346 inches (0.088 centimeters) center-to-center, have base diameters (BD) of .040 inches (0.120 centimeters), and hole diameters (HD) of .013—.014 inches (0.0330 to 0.0356 centimeters). The holes 141, Figure 11, have included angles A of 90 degrees, and included half angles B of 45 degrees. Thus, the perforated tubular member 122 of the preferred embodiment apparatus 40 has approximately four-hundred-thirty-five-thousand (435,000) holes 141 in it.

Still referring to Figures 10 and 11, it can be seen that particles 127 are affixed to tubular member 122 in any suitable manner. For purposes of illustration, the size of particles 127 is greatly enlarged. In a preferred method, tubular member 122 is cleaned thoughly with methyl ethyl ketone or vapor degreasing techniques in order to totally expose the base metal of tubular member 122. The cleaned tubular member 122 is coated with a mixture of particles 127 and epoxy which may be electrostatically sprayed onto tubular member 122 using any suitable spaying system such as is manufactured by Metokote Equipment Company of Lima, Ohio and marketed under the tradename Meco 10. Aluminum oxide particles were used and found to be satisfactory. After spraying the mixture of aluminum oxide particles and epoxy onto tubular member 122, the epoxy is cured.

By varying the ratio of particles 127 to epoxy, the texture of the film 51 is varied. Similarly, varying the size of particles 127 (as measured by the average particle diameter) will also vary the texture of film 51. Table I sets forth some of the aluminium oxide particle sizes and ratios of aluminium oxide particles to epoxy which were used and found to be satisfactory.

TABLE I

| Average particle diameter (mm) | 0.0635 | 0.057 | 0.049 | 0.041 |
|---|---|---|---|---|
| Ratios of particles to epoxy (by wt.) | 2.3:1 | 3:2 | 3:2 | 1.1:1 |

An acceptable epoxy is manufactured by Armstrong Products Company of Warsaw, Indiana and marketed under the tradename Armstrong Vibro-Flo E-7000 Series. Suitable aluminum oxide particles 127 are manufactured by General Abrasives Company of Niagara Falls, New York and are marketed under the tradename Lionblast. The aluminum oxide particles should be sorted by size which may be conveniently done by passing the particles 127 through a series of U.S. standard sieves such as those manufactured by W. S. Tyler Company of Cleveland, Ohio. Those particles which were found to work well had a diameter of from about 0.0015 to about 0.0030 inches (about 0.038 to about 0.076 mm.) and were found to produce nubbles 14 of the diameter within the ranges hereinbefore stated when the topsheet 16 thickness used was 0.001 inches (0.025 mm.).

Heaters 59 of the preferred apparatus, Figures 1 and 4, are Model No. 33-HS which are available from Kamweld Products Company, Inc., 90 Access Road, P.O. Box 91, Norwood, Mass. 02062. When operated on 120 volts, they provide 900 watts of heat and cause a flow of 2.83—3.77 dm³/sec to be heated approximately 243°C; substantially higher than the thermoplastic temperature of low density polyethylene.

The method of making perforated tubular member 122 is described in detail in the aforementioned U.S. patent application Serial No. 733,961.

To operate apparatus 40, the film 50 is looped, as shown in Figure 4, from roll 76, over idler roll 78, under dancer roll 80, over idler roll 78a, over the Mount Hope Vari-Bow roll 81, under the lead-on idler roll 65, clockwise about the texturing cylinder 55, under the lead-off/chill roll 66, through the nip 70 intermediate the lead-off chill roll 66 and the soft-face roll 67, over idler roll 78b, under dancer roll 94, over idler roll 78c, and thence onto a spool 200 on the roll winding shaft 201. The idler roll 78d is mounted so that it is gravitationally loaded against the top portion of the roll 90 being wound. The unwind tension control loop and the winding tension control loop are activated. The drive motor 202 is then operated by the drive controller 203 in response to operator inputs and in response to the feedback tachometer 204 to rotate the texturing cylinder 55 at a predetermined peripheral speed, and the chill roll 66 at a slightly higher (slack obviating) peripheral speed. The tension control loops respond to establish and maintain their respective predetermined levels of tension (as determined by the positions of weights 96) substantially irrespective of the film velocity.

Air is then directed through heaters 59, Figure 4, and electrical power is applied thereto whereby jets of hot air issue from the heaters 59 to form a virtual curtain of hot air directed radially inwardly towards sector SP, Figure 5, of the texturing cylinder 55. With sufficient levels of vacuum applied to the sectors ST and SC to isolate sector SP from upstream and downstream tension, vacuum applied to sector SP acts in concert with the hot air jets to texture, deboss/emboss and perforate the film.

When the preferred apparatus is used to texture, deboss/emboss and perforate low density polyethylene having a thickness of 0.025 mm at 15.25 metres per minute, the upstream tension is adjusted to 89.2 g/lineal centimetre, the downstream tension is adjusted to 22.3 g/linear centimetre, and the levels of vacuum applied to sectors ST, SP, and SC are 127, 381, and 127 mm of mercury respectively.

## Claims

1. A disposable absorbent structure comprising an absorbent body (30), a fluid impermeable backsheet (18) underlying the body and a macroscopically perforated thermoplastic film forming a topsheet (16) overlying the body, the body being encased by the backsheet and the topsheet characterised in that the perforated thermoplastic film is formed, over substantially the entire outer surface thereof with microscopic nubbles (14) dimensioned so as to leave the profile of the macroscopic perforations substantially unchanged.

2. A disposable absorbent structure according to Claim 1 characterised in that the nubbles (14) have a maximum cross sectional dimension of from 0.0127 mm to 0.279 mm and an average centre to centre spacing of from one to six times the maximum cross sectional dimension.

3. A disposable absorbent structure according to either one of Claims 1 and 2 characterised in that the nubbles (14) have a maximum cross-sectional dimension of from 0.025 to 0.178 mm preferably from 0.025 to 0.127 mm.

4. A disposable absorbent structure according to either one of Claims 2 and 3 characterised in that the nubbles (14) have an average centre to centre spacing of two to four times, preferably an average spacing of two to three times the maximum cross sectional dimension.

5. A disposable absorbent structure according to any one of Claims 2 to 4 characterised in that the nubbles (14) have an average height of at least 30% of their maximum cross-sectional dimension.

## Revendications

1. Structure absorbante à jeter comportant un corps absorbant (30), une contre-feuille imperméable aux fluides (18) sous-jacente au corps et une pellicule thermoplastique à perforations macroscopiques formant une feuille de dessus (16) recouvrant le corps, le corps étant enfermé par la contre-feuille et la feuille de dessus caractérisée en ce que la pellicule thermoplastique perforée présente, sur la quasi-totalité de sa surface extérieure des bosses microscopiques (14) ayant de telles dimensions qu'elles laissent le profil des perforations macroscopiques sensiblement inchangé.

2. Structure absorbante à jeter selon la revendication 1, caractérisée en ce que les bosses (14) ont une section maximale de 0,0127 mm à 0,279 mm et un espacement de centre à moyen égal à une à six fois la dimension de section maximale.

3. Structure absorbante jetable selon l'une ou l'autre des revendications 1 et 2, caractérisée en ce que les bosses (14) ont une section maximale de 0,025 à 0,178 mm de préférence de 0,025 à 0,127 mm.

4. Structure absorbante jetable selon l'une ou l'autre des revendications 2 et 3, caractérisée en ce que les bosses (14) ont un espacement de centre à centre moyen égal à deux à quatre fois, de préférence un espacement moyen égal à deux à trois fois la section maximale.

5. Structure absorbante à jeter selon l'une quelconque des revendications 2 à 4, caractérisée en ce que les bosses (14) ont une hauteur moyenne égale à au moins 30% de leur section maximale.

## Patentansprüche

1. Saugfähiger Gegenstand in Form eines Wegwerfartikels, umfassend einen saugfähigen Körper (30), eine unter dem Körper liegende, flüssigkeitsundurchlässige rückwärtige Lage (18) und eine über dem Körper liegende oberste Lage (16), die aus einer makroskopisch perforierten thermoplastischen Schicht gebildet ist, wobei der Körper von der rückwärtigen Lage und der obersten Lage umschlossen ist, dadurch gekennzeichnet, daß die perforierte thermoplastische Schicht über im wesentlichen ihre gesamte äußere Oberfläche mit mikroskopischen Noppen (14) ausgebildet ist, die so bemessen sind, daß sie das Profil der makroskopischen Perforationen im wesentlichen unverändert lassen.

2. Saugfähiger Gegenstand in Form eines Wegwerfartikels gemäß Anspruch 1, dadruch gekennzeichnet, daß die Noppen (14) eine maximale Querschnittsabmessung von 0,0127 mm bis 0,279 mm und einen durchschnittlichen Abstand von Zentrum zu Zentrum aufweisen, der 1 bis 6 mal der maximalen Querschnittsabmessung entspricht.

3. Saugfähiger Gegenstand in Form eines Wegwerfartikels gemäß einem der Ansprüche 1 und 2, dadurch gekennzeichnet, daß die Noppen (14) eine maximale Querschnittsabmessung von 0,025 bis 0,178 mm und vorzugsweise von 0,025 bis 0,127 mm aufweisen.

4. Saugfähiger Gegenstand in Form eines Wegwerfartikels gemäß einem der Ansprüche 2 und 3, dadurch gekennzeichnet, daß die Noppen (14) einen durchschnittlichen Abstand von Zentrum zu Zentrum aufweisen, der 2 bis 4 mal der maximalen Querschnittsabmessung entspricht, und vorzugsweise einen durchschnittlichen Abstand aufweisen, der 2 bis 3 mal der maximalen Querschnittsabmessung entspricht.

5. Saugfähiger Gegenstand in Form eines Wegwerfartikels gemäß einem der Ansprüche 2 bis 4, dadurch gekennzeichnet, daß die Noppen (14) eine durchschnittliche Höhe aufweisen, die mindestens 30 % ihrer maximalen Querschnittsabmessung entspricht.

# Fig. 1

# Fig. 2

# Fig. 3

1

Fig. 4

Fig. 5

0 018 684

## Fig. 6

## Fig. 7

## Fig. 8

4

# Fig. 9

# Fig. 11

# Fig. 10